# EUROPEAN PATENT SPECIFICATION

(11) **EP 0 787 482 B1**
(45) Date of publication and mention of the grant of the patent: **22.09.2004**
(21) Application number: 97300430.2
(22) Date of filing: 23.01.1997
(51) Int. Cl.: A61K 7/30

(54) **Denture cleansing compositions containing percompounds**
Gebissreinigungszusammensetzungen enthaltend Perverbindungen
Compositions pour le nettoyage des dentiers contenant des composés per

(30) Priority: 30.01.1996 GB 9601785
(43) Date of publication of application: 06.08.1997
(73) Proprietor: Kukident GmbH, D-69469 Weinheim (DE)
(72) Inventor: Knollmann, Rainer, 32052 Herford (DE); van de Loecht-Blasberg, Monika, 69469 Weinheim (DE); Rosenstengel, Michael, 67354 Romerberg (DE)
(74) Representative: Hall, Marina

(56) References cited:
- EP-A- 0 323 049
- EP-A- 0 400 858
- WO-A-94/26246
- US-A- 5 055 305
- Falbe J.; Regitz M.: 'Römpp Kompakt - Basislexikon Chemie', 1998, Georg Thieme Verlag, Deutschland, Seite 260.

## Description

The invention relates to a composition for use in denture cleansing and to denture cleansing tablets made therefrom.

It is sometimes necessary for humans to replace teeth which have been lost. Artificial teeth, which are implanted into the remaining jawbone, can be treated like ordinary teeth from the point of view of hygiene. In contrast, dentures must be specially treated to remove the accumulation of plaque and mucilageneous and bacterial deposits which collect while the denture is being worn. This is also the case for braces being worn by many children nowadays.

Denture cleanser products such as effervescence tablets and powders are well known in the art.

Denture cleansing tablets are known which consist of two phases. The first phase of such tablets is responsible for conditioning the medium and providing an oxygen-free cleansing procedure step. The second phase is responsible for a second cleansing step making use of oxygen, thereby oxidizing the primary and secondary plaque already attacked in the first step of the cleansing procedure mediated by the first phase of the denture cleansing tablet.

Typically, two-phase cleansing systems are generated by two-layer tablets or the like, which impose tremendous problems to the manufacturer, as during the manufacturing process of such denture cleansing tablets, a first layer has to be created which is hard enough to survive as a pre-tablet and yet soft enough to allow key-binding of the following second layer which is pressed on the first one. To fulfill these special requirements, finely balanced formulations and respective pressure are needed, which is disadvantageous with regard to cost and environmental performance.

To date, mostly perborates or persulphates have been used as inorganic persalt bleaching agents within compositions for use in denture cleansing. Despite an acceptable cleansing performance, perborate causes some problems with regard to its solubility when dissolving a composition comprising perborate in water. Additionally, the environmental performance of perborate is not particularly good. The same is true for persulphate.

WO 94/26246 discloses denture cleansing tablets comprising visually discrete agglomerated particles of an organic peroxyacid bleach precursor dispersed in a water-soluble or water dispersible matrix comprising an inorganic persalt bleaching agent and a solid base material which in the presence of water releases carbon dioxide or oxygen with effervescence.

EP 0 400 858 discloses granular denture cleansing compositions comprising an inorganic persalt bleaching agent, an organic peroxyacid bleach precursor and an effervescence generator.

We have now devised a composition for use in denture cleansing that reduces or overcomes the disadvantages of known denture cleansing compositions.

In particular, the present invention provides an improved composition for use in denture cleansing that shows excellent cleansing properties, including germ killing ability, and exhibits better solubility properties than known compositions.

Furthermore, the present invention also provides a composition for use in denture cleansing having a better environmental performance.

According to one aspect of the invention, there is provided a composition for use in denture cleansing comprising at least one organic peroxyacid bleach precursor, at least one inorganic persalt bleaching agent and a water-soluble or water-dispersable matrix comprising a solid base material which in the presence of water releases carbon dioxide and/or oxygen with effervescence, with the solid base material comprising at least one component phase having an acid or a neutral pH in aqueous medium and at least one inorganic persalt bleaching agent incorporated therein being a percarbonate, wherein the percarbonate is used as and/or is present as a particulate fraction.

The invention also provides a denture cleansing tablet that contains a composition according to the present invention.

Furthermore, the invention provides the use of the composition of the invention in a denture cleansing process.

The invention further provides the use of the composition of the invention to remove plaque and/or stains from teeth and/or to prevent plaque and/or stains from forming on teeth.

Furthermore, the invention provides the use of the composition of the invention in the manufacture of a medicament to remove plaque and/or stains from teeth and/or to prevent plaque and/or stains from forming on teeth.

Preferably, the percarbonate is an alkali metal percarbonate, an alkaline earth metal percarbonate or a mixture thereof.

It is preferred that the percarbonate is used as and/or is present as visible discrete entities.

In a preferred embodiment of the invention, the particulate fraction or visible discrete entities further comprise at least part of the overall content of the organic peroxyacid bleach precursor.

Preferably, the particulate fraction or visible discrete entities further comprise at least one enzyme.

In a further embodiment of the present invention the composition comprises at least one enzyme which is incorporated into the water-soluble or water-dispersable matrix.

Preferably, at least one enzyme is incorporated into the solid base material.

In another embodiment of the present invention, the composition comprises at least one enzyme which is incorporated into the component phase having an acid or neutral pH in aqueous medium.

Preferably, the inventive composition comprises at least one enzyme with said enzyme being used as and/or being present as a particulate fraction and/or visible discrete entities. More preferably, the particulate fraction and/or visible discrete entities further comprise(s) at least part of the overall content of the organic peroxyacid bleach precursor.

In a preferred embodiment the enzyme is a plaque and/or stain removing and/or preventing enzyme.

Preferably, the enzyme is a protease, lipase or glycosidase. Suitable proteases are pepsin, papain, trypsin, acidic proteases, and alkali proteases, for example. A suitable glycosidase is.a glucanase, for example, an endoglucanase such as an amylase or an exoglucanase.

Preferably, the enzyme is produced by genetic engineering techniques. Alternatively, the enzyme may be isolated from biological material originally producing said enzymatic activity.

In accordance with the present invention, the composition can contain a mixture of at least two enzymatic activities. Furthermore, it can be useful that the composition contains further proteinaceous material.

The enzyme may be of animal origin and/or plant origin and/or microbial origin.

In an embodiment of the present invention, at least part of the total amount of the organic peroxyacid bleach precursor is used as and/or is present as a particulate fraction. Preferably, at least part of the total amount of the organic peroxyacid bleach precursor is used as and/or is present as visible discrete entities.

In a further embodiment, at least part of the total amount of the organic peroxyacid bleach precursor may be present in the matrix.

Preferably, the particulate fraction or visible discrete entities containing the organic peroxyacid bleach precursor has/have an alkaline pH in aqueous medium. Alternatively, the particulate fraction or visible discrete entities containing the organic peroxyacid bleach precursor, has/have an acid pH in aqueous medium.

In accordance with the present invention, the particulate fraction or visible discrete entities may contain an effervescence generator.

In a preferred embodiment of the composition of the invention the solid base material may comprise a (bi)carbonate/acid effervescent couple, wherein at least the component phase having an acid or neutral pH contains both components of the effervescent couple.

Preferably, the organic peroxyacid bleach precursor is selected from acylated polyalkyldiamines, especially tetraacetylethylenediamine, and carboxylic esters having the general formula AcL wherein Ac is the acyl moiety or an organic carboxylic acid comprising an optionally substituted, linear or branched C₆-C₂₀ alkyl or alkenyl moiety or a C₆-C₂₀ alkyl-substituted aryl moiety and L is a leaving group, the conjugate acid of which has a pKa in the range from 4 to 13.

In an embodiment of the present invention, the composition further comprises a flavouring agent, an agent for plaque removal, tartar removal, plaque control, tartar control, surface modification, natural teeth preservation, saliva regulation, bad breath neutralization, freshness, an anti-soil/anti-bacterial agent or a mixture of two or more thereof.

In a preferred embodiment of the inventive composition at least one of said agents or any combination of said agents is used and/or is present as an additional particulate fraction or visible discrete entities.

In another preferred embodiment of the present invention at least one of said agents or any combination of said agents forms part of any particulate fraction of the composition and/or any visible discrete entities contained therein. Alternatively, at least one of said agents or any combination of said agents is present in the matrix.

A composition for use in denture cleansing in accordance with the present invention is advantageous for several reasons.

For example, the use of percarbonate reduces the problems arising from the low solubility of other conventional inorganic persalt bleaching agents, which has resulted in the need for sophisticated formulations of cleansing compositions and has thus imposed disadvantageous restrictions on the choice of the compounds used in the overall composition.

These problems are overcome by the utilization of percarbonate as inorganic persalt bleaching agent, thus also making the manufacturing of the compositions of the invention easier and less expensive.

A further advantage of the inventive composition is its improved overall environmental performance based on the utilization of percarbonate. Some other inorganic persalt neutralization, freshness, an anti-soil/anti-bacterial agent or a mixture of two or more thereof.

In a preferred embodiment of the inventive composition at least one of said agents or any combination of said agents is used and/or is present as an additional particulate fraction or visible discrete entities having a diameter of 150-1000µm.

In another preferred embodiment of the present invention at least one of said agents or any combination of said agents forms part of any particulate fraction of the composition and/or any visible discrete entities having a diameter of 150-1000µm contained therein. Alternatively, at least one of said agents or any combination of said agents is present in the matrix.

A composition for use in denture cleansing in accordance with the present invention is advantageous for several reasons.

For example, the use of percarbonate reduces the problems arising from the low solubility of other conventional inorganic persalt bleaching agents, which has resulted in the need for sophisticated formulations of cleansing compositions and has thus imposed disadvantageous restrictions on the choice of the compounds used in the overall composition.

These problems are overcome by the utilization of percarbonate as inorganic persalt bleaching agent, thus also making the manufacturing of the compositions of the invention easier and less expensive.

A further advantage of the inventive composition is its improved overall environmental performance based on the utilization of percarbonate. Some other inorganic persalt bleaching agents are not that easily metabolized by a variety of microorganisms, or are even toxic to certain animals such as fish, thus imposing additional stress to the environment, both during their manufacture as well as their deposition after usage.

Additionally, it is advantageous to use a particulate fraction containing the percarbonate or corresponding visible discrete entities because it/they allow for a controlled use of the percarbonate forming it/them or being enclosed or associated therewith at the level of the individual particle or visible discrete entity rather than at the level of a complete layer incorporating the percarbonate.

The releasing kinetics of the percarbonate containing particulate fraction or particles from the acidic or neutral component phase can be influenced is many different ways, such as, for example, by diverse coatings, use of salts such as sodium chloride and sodium sulphate, or compounds which are rapidly or slowly soluble or by using fillers or polymers such as proteins, cellulose ethers, cellulose esters, polyvinyl alcohol, alginic acid esters, or other polymers having a colloidal or pseudo-colloidal character.

Another advantage of the inventive composition which arises from the percarbonate being used as and/or being present as a particulate fraction or visible discrete entities is that it is possible to monitor the correct distribution of the particulate fraction or the visible discrete entities in the matrix. This is of great importance for the manufacturing process. Because of the consistently high quality which can thus be guaranteed, the efficacy of a denture cleansing process which makes use of said inventive composition is increased.

Further beneficial effects with regard to cleansing efficacy arise when the particulate fraction or visible discrete entities further comprise at least part of the overall content of the organic peroxyacid bleach precursor, due to the close proximity of the organic peroxyacid bleach precursor on the one hand and the inorganic persalt bleaching agent, i.e. percarbonate, on the other hand.

It must be clear that, from the point of view of stability, certain combinations of compounds in a particle, particulate fraction or visible discrete entities of the inventive composition are more preferred than others. For example combining TAED as a representative organic peroxyacid bleach precursor with any enzyme in a particle can be realized without any problems. In contrast, it is sometimes necessary to enhance the stability of a combination of an inorganic persalt bleaching agent and an organic peroxyacid bleach precursor in a single particle or the combination of an inorganic persalt bleaching agent and an enzyme in a single particle. For example, suitable coatings are available or appropriate fillers can be used to create a particle which does not allow any direct contact of the compounds with each other, i.e. a compartmentalized particle or entity, which nevertheless exhibits the above-mentioned benefits arising from particles, regardless of whether they are present as particulate fractions or visible discrete entities.

This positive effect can also be observed when other composition compounds are combined within a particulate fraction and/or visible discrete entities and is mostly based on any conditioning of the medium which allows further components to develop their optimum performance.

The use of enzymes within the inventive composition confers further advantages thereto, such as specific and non-aggressive plaque and/or stain removal and/or plaque and/or stain prevention, thus erasing the basis of many undesirable effects arising from inadequate denture cleansing.

The advantages of having one or more composition compounds, separately or combined, present as or used as a particulate fraction or as visible discrete entities, as discussed above, are also true for any enzyme or organic peroxyacid bleach precursor, many of which are known in literature, contained in the inventive composition.

A further advantage of the fact that the enzyme particles are visible discrete entities is that it is possible to mask the undesirable colour of the enzymes per se. Such undesirable colour of the enzymes or of the respective enzyme preparations might arise from coenzymes or any contaminants. This is of special importance because of aesthetic considerations.

Because of the tremendous variety of enzymes, for most material which is detrimental to dentures, appropriate enzymatic activity can be found for cleansing or supporting the cleansing procedure of the denture. It is the diversity of enzymes of animal origin and/or plant origin and/or microbial origin, allowing their use under various ionic strength and pH levels, that make them a very specific tool in removing plaque and/or stains and/or preventing further accumulation thereof. With regard to the chemical nature of many materials which are detrimental to dentures, acidic proteases, alkali proteases and amylases, such as, for example, alpha-amylases, have been proven to be especially advantageous.

The use of an enzyme produced by genetic engineering techniques may be beneficial from a financial point of view, as said techniques allow a cheap production of the desired enzymatic activity to be added to the composition.

The wording "enzymes being produced by genetic engineering techniques" is intended to include also those enzymes that have been modified by such techniques to make them more resistant to the chemical composition to which they are to be exposed. For example, other enzymatic activities contained in the inventive composition may attack the protein chain of the added enzymatic activity, thus limiting its efficacy. On the other hand, by introducing cleavage sites for which other added enzymatic activities are specific, a stringent control of the enzymatic activities is possible.

Alternatively, an enzyme isolated from biological material originally producing said enzymatic activity might also be of advantage, especially with regard to the fact that no cloning or the like is necessary and an abundant source for the respective enzymatic activity allows easy access to said enzymatic activity. Furthermore, such enzyme preparations gained from biological material may possibly comprise a mixture of enzymatic activities that act in a synergistic manner, thus improving the overall denture cleansing and sanitizing process.

The synergistic effect and the stringent control of the enzymatic activities may especially result from a mixture of at least two enzymatic activities being incorporated in one single type of particles, possibly with the particles being dispersed in the matrix and being visible discrete entities.

It is not necessary that the enzyme preparation used in the inventive composition has been isolated to homogeneity, although such an enzyme preparation might also advantageously be used with regard to avoidance of allergic reactions and to quality control. Enzyme preparations comprising further proteinaceous material are also useful because a cheaper production of the enzyme preparation is possible, as it is less time-consuming and additionally any loss of enzyme activity resulting from further purification steps is avoided. In addition, the further proteinaceous material can act as a filler to stabilize the particles containing at least one plaque and/or stain removing and/or preventing enzyme being visible discrete entities and being dispersed in the matrix so as to control the release of said particles from the water-soluble or water-dispersable matrix and/or of the enzyme itself from said particles.

The enzyme excercises its beneficial effect in the denture cleansing procedure under circumstances that are most appropriate for its action, for example an alkali protease is released under an alkaline pH to, for example, remove food debris attached to the teeth.

To further improve the overall cleansing performance of the inventive composition, the organic peroxyacid bleach precursor containing particulate fraction or visible discrete entities, possibly containing other compounds such as enzymes or percarbonate, can have an alkaline pH in aqueous medium. Because of this alkaline pH, possibly supported by another component phase contained within the solid base material of the inventive composition, a second phase of the cleansing process can be established which is especially advantageous after an acid pH cleansing step or phase. Furthermore the alkaline pH can provide appropriate pH conditions for another enzymatic activity contained in the inventive composition.

Designing the particulate fraction or visible discrete entities containing the organic peroxyacid bleach precursor so as to have an acid pH in aqueous medium further supports the cleansing performance as the acid pH, possibly also generated by the appropriate component phase of the solid base material which also contains the percarbonate, is known as a preferred germ killing condition. Additionally, the acid pH is more appropriate with regard to solution clarity, which is an important factor because of aesthetic considerations which will be taken into account by the consumer.

The particulate fraction and/or visible discrete entities of the inventive composition, whatever its/their content may be, further comprising an effervescence generator is/are especially useful to achieve a good cleansing performance, as said generator makes sure that the fraction(s) or entities is/are moved relatively to the aqueous medium, thus avoiding any gradient formation and making sure that no problems arising from transport limitations or the like reduce the efficacy of the cleansing process.

The incorporation of an effervescence generator refers to particulate fraction(s), visible discrete entities or the like containing at least one enzyme as well as to those containing percarbonate or peroxyacid bleach precursors or those containing at least two of said compounds. Besides this physical effect, the chemical effects arising from the effervescence generators are beneficial as they provide at least a micro-environment that is suitable for the optimum action of the compounds they are associated with. This includes the presence of oxygen for further attacking, for example, the food debris already attacked by the enzyme, or the other way round, preparing the food debris to be attacked by the enzymatic activity, as well as the generation of carbon dioxide being, for example, responsible for an effective dissolution of the composition and mechanical cleansing effects, thus further increasing the overall cleansing efficacy.

Effervescence generators which are effective under alkaline pH conditions include persalts such as alkali and alkaline earth metal peroxyborates as well as sodium perborate anhydrous, persulphates, percarbonates, perphosphates and mixtures of two or more thereof.

Effervescence generators which are effective under acid or neutral pH conditions include a combination of at least one alkali metal carbonate or bicarbonate, such as sodium bicarbonate, sodium carbonate, sodium sesquicarbonate, potassium carbonate, potassium bicarbonate or mixtures of two or more thereof in admixture with at least one non-toxic, physiologically acceptable organic acid, such as tartaric, fumaric, citric, malic, maleic, gluconic, succinic, salicylic, adipic or sulphamic acid, sodium fumarate, sodium or potassium acid phosphates, betaine hydrochlorides or mixtures of two or more thereof.

When the solid base material comprises a (bi)carbonate/acid effervescent couple and at least the component phase has an acid or neutral pH containing both components of the effervescent couple, further improvement of the mixing within the aqueous medium is possible, thus in turn overcoming transport limitations and therefore increasing cleansing efficacy.

The advantages as outlined in the above paragraphs are also true for compositions which also contain flavouring agents, agents for plaque removal, tartar removal, plaque control, tartar control, surface modification, natural teeth preservation, saliva regulation, bad breath neutralization, freshness, an anti-soil/anti-bacterial agent or a mixture of two or more thereof. If at least one of said agents or any combination thereof is used and/or is present as an additional particulate fraction or visible discrete entities, the advantages as set forth for other particulate fractions or visible discrete entities according to the present invention are given, too, including the situation that at least one of said agents or any combination of said agents forms part of any other particulate fraction of the composition and/or any visible discrete entities contained therein.

In a preferred embodiment of the invention, the composition comprises about 0.5 to 5 wt % organic peroxyacid bleach precursor, about 10 to 40 wt % inorganic persalt bleaching agent and about 55 to 89.5 wt % matrix. Optionally, the composition comprises 0.5 to 5 wt % organic peroxyacid bleach precursor, about 10 to 40 wt % inorganic persalt bleaching agent and also comprises enzyme, preferably in an amount of about 0.5 to 5 wt %, and further additives such as flavouring agents and agents for plaque removal, with the balance being matrix.

Most preferably, the amount of organic peroxyacid bleach precursor is about 0.5 to 3 wt %, and in particular about 1 wt % and the amount of inorganic persalt bleaching agent is about 15 to 35 wt %, and in particular about 23 wt %.

In order that the invention may be more fully understood, the following examples will now be described, by way of illustration only.

### Examples

Table 1 sets forth some compositions according to the present invention. All amounts given are percent by weight.

**Table 1**

| Ingredient | Ex. 1 | Ex. 2 | Ex. 3 | Ex. 4 |
|---|---|---|---|---|
| Potassium monopersulphate | 20,00 | 20,00 | 20,00 | 20,00 |
| Sodium percarbonate | 3,0 | 3,0 | 3,0 | 3,0 |
| TAED | 1,00 | 1,00 | 1,00 | 1,00 |
| Sodium bicarbonate | 20,00 | 20,00 | 20,00 | 20,00 |
| Sodium carbonate | 10,00 | 10,00 | 10,00 | 10,00 |
| Citric acid anhydrous | 10,00 | 10,00 | 10,00 | 10,00 |
| Malic acid | 10,00 | 10,00 | 10,00 | 10,00 |
| Sulfamic acid | 3,00 | 3,00 | 3,00 | 3,00 |
| Sodium sulphate anhydrous | 11,99 | 11,99 | 11,99 | 8,99 |
| Sodium dodecyl benzene sulphonate | 0,50 | 0,50 | 0,50 | 0,50 |
| Flavour | 3,00 | 3,00 | 3,00 | 3,00 |
| PEG 4000 | 0,70 | 0,70 | 0,70 | 0,70 |
| PEG 6000 | 5,30 | 5,30 | 5,30 | 5,30 |
| Dyestuff | 0,01 | 0,01 | 0,01 | 0,01 |
| Coloured Protease like Novozym® 243 | 1,50 | - | - | 1,50 |
| Coloured Amylase like Termamyl ® | - | 1,50 | - | 1,50 |
| Coloured Lipase like Lipozyme™ | - | - | 1,50 | 1,50 |
| Total | 100 | 100 | 100 | 100 |
| Ex: Example | | | | |

Among the above-mentioned ingredients, sodium percarbonate and potassium monopersulphate are the inorganic persalt bleaching agents, and thus the oxidizing agents supplying active oxygen to the solution.

TAED is an organic peroxyacid bleach precursor.

Sodium bicarbonate serves as a gaseous carbon dioxide supply, if supplied with organic acids. The gaseous carbon dioxide influences the dissolution characteristics of the composition and bubbles of carbon dioxide effect mechanical cleansing.

The organic acids are part of the effervescence system, serve as complexing agents and are involved in the regulation of the pH.

Sodium dodecyl benzene sulphonate is a detergent improving cleansing activity.

PEG 6000 and PEG 4000 are tabletting aids.

Sodium carbonate is part of the pH regulation system and a stability aid.

Sodium sulphate acts as a filler.

Preferably, the enzyme containing particles have a diameter of 150 to 1000 µm. The enzyme particles comprise a coating consisting of sodium chloride, sodium sulphate or combinations thereof. Other compounds such as PEG might also be contained within said particles which serve as a binder. The enzyme containing particles are of red, green or blue colour.

## Claims

1. A composition for use in denture cleansing comprising at least one organic peroxyacid bleach precursor, at least one inorganic persalt bleaching agent and a water-soluble or water-dispersable matrix comprising a solid base material which in the presence of water releases carbon dioxide and/or oxygen with effervescence, with the solid base material comprising at least one component phase having an acid or a neutral pH in aqueous medium and at least one inorganic persalt bleaching agent incorporated therein being a percarbonate, wherein the percarbonate is used as and/or is present as a particulate fraction and/or as visible discrete entities.

2. A composition according to claim 1, wherein the percarbonate is an alkali metal percarbonate, an alkaline earth metal percarbonate or a mixture thereof.

3. A composition according to claim 1 or 2, wherein said particulate fraction or visible discrete entities further comprise(s) at least part of the overall content of the organic peroxyacid bleach precursor and/or at least one enzyme.

4. A composition according to claims 1, 2 or 3, wherein at least part of the total amount of the organic peroxyacid bleach precursor is used as and/or is present as a particulate fraction, preferably as visible discrete entities, and/or is present in the matrix.

5. A composition according to any of claims 1 to 4, with the organic peroxyacid bleach precursor being selected from acylated polyalkyldiamines, especially tetraacetylethylenediamine, and carboxylic esters having the general formula AcL wherein Ac is the acyl moiety or an organic carboxylic acid comprising an optionally substituted, linear or branched C₆-C₂₀ alkyl or alkenyl moiety or a C₆-C₂₀ alkyl-substituted aryl moiety and L is a leaving group, the conjugate acid of which has a pKa in the range from 4 to 13.

6. A composition according to any of claims 1 to 5, wherein an enzyme is incorporated into the solid base material and/or into the component phase having an acid or neutral pH in aqueous medium and/or is present as a particulate fraction and/or visible discrete entities.

7. A composition according to claims 3 to 6, wherein the enzyme is selected from the group comprising proteases, lipases, and glycosidases, with the protease being more particularly selected from the group comprising pepsin, papain, trypsin, acidic proteases or alkali proteases, and the glycosidase being preferably a glucanase and more preferably an endoglucanase or exoglucanase and most preferably an amylase.

8. A composition according to any of claim 1 to 7, wherein any of the particulate fraction or visible discrete entities contain(s) an effervescence generator.

9. A composition according to any of claims 1 to 8, further comprising a flavouring agent, an agent for plaque removal, tartar removal, plaque control, tartar control, surface modification, natural teeth preservation, saliva regulation, bad breath neutralization, freshness, an anti-soil/anti-bacterial agent or a mixture of two or more thereof, and preferably at least one of said agents or any combination of said agents is used and/or is present as an additional particulate fraction or visible discrete entities and/or forms part of any particulate fraction of the composition and/or any visible discrete entities contained therein.

10. A denture cleansing tablet containing a composition according to any of claims 1 to 9.

## Patentansprüche

1. Zusammensetzung zur Verwendung in einer Zahnprothesenspülung, umfassend mindestens eine Vorstufe aus einer organischen Peroxysäurenbleiche, mindestens ein Bleichmittel aus einem anorganischen Persalz und eine wasserlösliche oder wasserdispergierbare Matrix, die ein festes Grundmaterial umfasst, das in Gegenwart von Wasser unter Sprudeln Kohlendioxid und/oder Sauerstoff freisetzt, wobei das feste Grundmaterial mindestens eine Komponentenphase mit saurem oder neutralem pH-Wert in wässrigem Medium und mindestens ein darin eingebrachtes Bleichmittel aus einem anorganischen Persalz, das ein Percarbonat ist, umfasst, wobei das Percarbonat als teilchenförmige Fraktion und/oder sichtbar getrennte Einheiten verwendet wird und/oder vorliegt.

2. Zusammensetzung nach Anspruch 1, wobei es sich bei dem Percarbonat um ein Alkalimetallcarbonat, ein Erdalkalimetallcarbonat oder ein Gemisch davon handelt.

3. Zusammensetzung nach Anspruch 1 oder 2, wobei die teilchenförmige Fraktion oder die sichtbar getrennten Einheiten ferner zumindest einen Teil des Gesamtgehalts der Vorstufe aus einer organischen Peroxysäurenbleiche und oder mindestens ein Enzym umfasst oder umfassen.

4. Zusammensetzung nach den Ansprüchen 1, 2 oder 3, wobei zumindest ein Teil der Gesamtmenge der Vorstufe aus einer organischen Peroxysäurenbleiche als teilchenförmige Fraktion, vorzugsweise als sichtbar getrennte Einheiten verwendet wird und/oder vorliegt und/oder in der Matrix vorliegt.

5. Zusammensetzung nach einem der Ansprüche 1 bis 4, wobei die Vorstufe aus einer organischen Peroxysäurenbleiche ausgewählt ist aus acylierten Polyalkyldiaminen, insbesondere Tetraacetylethylendiamin, und Carbonsäureestem der allgemeinen Formel AcL, wobei Ac die Acyleinheit oder eine anorganische Carbonsäure, umfassend eine gegebenenfalls substituierte, lineare oder verzweigte C₆-C₂₀-Alkyl- oder -Alkenyleinheit oder eine C₆-C₂₀-alkylsubstituierte Aryleinheit, ist und L eine austretende Gruppe ist, wobei die Konjugatsäure davon einen pKa-Wert im Bereich von 4 bis 13 aufweist.

6. Zusammensetzung nach einem der Ansprüche 1 bis 5, wobei ein Enzym in das feste Grundmaterial und/oder in die Komponentenphase mit saurem oder neutralem pH-Wert in wässrigem Medium eingebracht ist und/oder als teilchenförmige Fraktion und/oder sichtbar getrennte Einheiten vorliegt.

7. Zusammensetzung nach den Ansprüchen 3 bis 6, wobei das Enzym ausgewählt ist aus der Gruppe, umfassend Proteasen, Lipasen und Glycosidasen, wobei die Protease insbesondere ausgewählt ist aus der Gruppe, umfassend Pepsin, Papain, Trypsin, saure Proteasen oder alkalische Proteasen, und die Glycosidase vorzugsweise eine Glukanase und stärker bevorzugt eine Endoglucanase oder Exoglucanase und besonders bevorzugt eine Amylase ist.

8. Zusammensetzung nach einem der Ansprüche 1 bis 7, wobei jede der teilchenförmigen Fraktion oder alle der sichtbar getrennten Einheiten einen Sprudelbildner enthält oder enthalten.

9. Zusammensetzung nach einem der Ansprüche 1 bis 8, ferner umfassend einen Geschmackstoff, ein Mittel zur Plaquentfernung, Zahnsteinentfernung, Plaquebekämpfung, Zahnsteinbekämpfung, Oberflächenmodifikation, für den Schutz natürlicher Zähne, zur Speichelregulierung, Neutralisation von schlechtem Atem, für Frische, ein Antiverschmutzungsmittel/antibakterielles Mittel oder ein Gemisch aus zwei oder mehreren davon umfasst und vorzugsweise mindestens eines der Mittel oder eine beliebige Kombination aus den Mitteln als zusätzliche teilchenförmige Fraktion oder sichtbar getrennte Einheiten verwendet wird oder vorliegt und/oder einen Teil einer beliebigen teilchenförmigen Fraktion der Zusammensetzung und/oder von beliebigen darin enthaltenen sichtbar getrennten Einheiten bildet.

10. Zahnprothesespülungstablette, enthaltend eine Zusammensetzung nach einem der Ansprüche 1 bis 9.

## Revendications

1. Composition destinée à être utilisée pour le nettoyage de prothèses dentaires, comprenant au moins un précurseur d'agent de blanchiment du type peroxyacide organique, au moins un agent de blanchiment du type persel inorganique et une matrice hydrosoluble ou dispersable dans l'eau comprenant une matière solide de base qui, en présence d'eau, libère du dioxyde de carbone et/ou de l'oxygène avec une effervescence, la matière soluble de base comprenant au moins une phase constitutive ayant un pH acide ou un pH neutre dans un milieu aqueux et au moins un agent de blanchiment du type persel inorganique incorporé à cette matière, qui consiste en un percarbonate, ledit percarbonate étant utilisé et/ou étant présent sous forme d'une fraction en particules et/ou sous forme d'entités discrètes visibles.

2. Composition suivant la revendication 1, dans laquelle le percarbonate est un percarbonate de métal alcalin, un percarbonate de métal alcalinoterreux ou un de leurs mélanges.

3. Composition suivant la revendication 1 ou 2, dans laquelle ladite fraction en particules ou lesdites entités discrètes visibles comprennent en outre au moins une partie de la quantité totale du précurseur d'agent de blanchiment du type peroxyacide organique et/ou au moins une enzyme.

4. Composition suivant la revendication 1, 2 ou 3, dans laquelle au moins une partie de la quantité totale du précurseur d'agent de blanchiment du type peroxyacide organique est utilisée et/ou est présente sous forme d'une fraction en particules, de préférence sous forme d'entités discrètes visibles, et/ou est présente dans la matrice.

5. Composition suivant l'une quelconque des revendications 1 à 4, dans laquelle le précurseur d'agent de blanchiment du type peroxyacide organique est choisi entre des polyalkyldiamines acylées, notamment la tétraacétyléthylène-diamine, et des esters carboxyliques répondant à la formule générale AcL dans laquelle Ac représente le groupement acyle ou un acide carboxylique organique comprenant un groupement alkyle ou alcényle en C₆ à C₂₀ linéaire ou ramifié, facultativement substitué, ou un groupement aryle à substituant alkyle en C₆ à C₂₀ et L représente un groupe partant, dont l'acide conjugué a un pKa compris dans la plage de 4 à 13.

6. Composition suivant l'une quelconque des revendications 1 à 5, dans laquelle une enzyme est incorporée à la matière solide de base et/ou à la phase constitutive ayant un pH acide ou neutre dans un milieu aqueux et/ou est présente sous forme d'une fraction en particules et/ou d'entités discrètes visibles.

7. Composition suivant les revendications 3 à 6, dans laquelle l'enzyme est choisie dans le groupe comprenant des protéases, des lipases et des glycosidases, la protéase étant choisie plus particulièrement dans le groupe comprenant la pepsine, la papaïne, la trypsine, des protéases acides ou des protéases alcalines, et la glycosidase étant avantageusement une glucanase et plus avantageusement une endoglucanase ou exoglucanase et de préférence une amylase.

8. Composition suivant l'une quelconque des revendications 1 à 7, dans laquelle n'importe quelle fraction en particules ou n'importe quelles entités discrètes visibles contiennent un agent engendrant une effervescence.

9. Composition suivant l'une quelconque des revendications 1 à 8, comprenant en outre un agent aromatisant, un agent pour l'élimination de la plaque, l'élimination du tartre, la lutte contre la plaque, la lutte contre le tartre, une modification de surface, la préservation des dents naturelles, la régulation de la salive, la neutralisation de la mauvaise haleine, la fraîcheur, un agent anti-salissures/antibactérien ou un mélange de deux ou plus de deux de ces agents, et de préférence au moins un desdits agents ou n'importe quelle association desdits agents est utilisé(e) et/ou est présent(e) sous forme d'une fraction en particules supplémentaire ou d'entités discrètes visibles supplémentaires et/ou forme une partie de n'importe quelle fraction en particules de la composition et/ou de n'importe quelles entités discrètes visibles présentes dans cette composition.

10. Tablette pour le nettoyage des prothèses dentaires, contenant une composition suivant l'une quelconque des revendications 1 à 9.
